(19) Europäisches Patentamt
European Patent Office
Office européen des brevets

(11) Veröffentlichungsnummer: **0 182 187**
**A1**

# (12) EUROPÄISCHE PATENTANMELDUNG

(21) Anmeldenummer: **85114018.6**

(22) Anmeldetag: **04.11.85**

(51) Int. Cl.⁴: **A 61 K 7/13**

(30) Priorität: **10.11.84 DE 3441148**

(43) Veröffentlichungstag der Anmeldung: **28.05.86**
**Patentblatt 86/22**

(84) Benannte Vertragsstaaten: **AT BE CH DE FR GB IT LI LU NL SE**

(71) Anmelder: **Wella Aktiengesellschaft, Berliner Allee 65, D-6100 Darmstadt (DE)**

(72) Erfinder: **Clausen, Thomas, Dr., Ernst Pasqué Strasse 35 A, D-6146 Alsbach (DE)**
Erfinder: **Konrad, Eugen, Mecklenburger Strasse 101, D-6100 Darmstadt (DE)**

## (54) Oxidationshaarfärbemittel auf der Basis von 4-Amino-2-hydroxyalkyl-phenolen.

(57) Oxidationshaarfärbemittel auf der Basis von 4-Amino-2-hydroxyalkyl-phenolen der allgemeinen Formel I

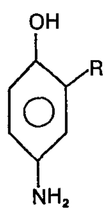

(I),

wobei R einen Monohydroxyalkylrest mit 1 bis 4 Kohlenstoffatomen bedeutet, oder dessen Salz als Entwicklersubstanz. Gegenstand der Anmeldung ist ferner die neue Entwicklersubstanz 4-Amino-2-(2'-hydroxyethyl)-phenol. Die Entwicklersubstanzen der Formel I weisen, bei gleich gutem färberischen Verhalten, physiologisch bessere Eigenschaften auf als das für Färbungen im Rotbereich bisher hauptsächlich verwendete p-Aminophenol.

EP 0 182 187 A1

Oxidationshaarfärbemittel auf der Basis von 4-Amino-2-hydroxyalkyl-phenolen

Gegenstand der Erfindung sind Mittel zur oxidativen Färbung von Haaren auf der Basis von 4-Amino-2-hydroxyalkyl-phenolen als Entwicklersubstanz sowie neue 4-Amino-2-hydroxyalkyl-phenole.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, 2,5-Diaminobenzylalkohol, p-Aminophenol und 1,4-Diaminobenzol eingesetzt. Von den vorzugsweise verwendeten Kupplersubstanzen sind Resorcin, 4-Chlorresorcin, 1-Naphthol, m-Aminophenol und Derivate des m-Phenylendiamins zu nennen.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombinationen geeigneter Entwickler- und Kupplerkomponenten eine breite Palette verschiedener Farbnuancen erzeugt werden kann. Zur Erzielung natürlicher und besonders modischer Nuancen im Rotbereich

wird vor allem p-Aminophenol, allein oder im Gemisch mit anderen Entwicklersubstanzen, in Kombination mit geeigneten Kupplersubstanzen eingesetzt.

Gegen den für den Rotbereich der Farbskala bisher hauptsächlich eingesetzten Entwickler p-Aminophenol werden in letzter Zeit Bedenken in Bezug auf die physiologische Verträglichkeit erhoben, während die in neuerer Zeit empfohlenen Entwicklersubstanzen, wie z. B. Pyrimidinderivate, in färberischer Hinsicht nicht völlig zufrieden - stellen können.

Es bestand daher die Aufgabe, Oxidationshaarfärbemittel auf der Basis von Entwicklersubstanzen für den Rotbe - reich aufzufinden, die den erwähnten Anforderungen besser gerecht werden.

Hierzu wurde nun gefunden, daß durch Mittel zur oxidatiben Färbung von Haaren auf der Basis einer Entwicklersubstanz-Kupplersubstanz-Kombination, dadurch gekennzeichnet, daß sie als Entwicklersubstanz ein 4-Amino-2-hydroxyalkylphenol der allgemeinen Formel I

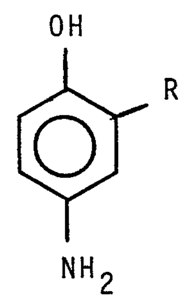

(I),

wobei R einen Monohydroxyalkylrest mit 1 bis 4 Kohlen - stoffatomen bedeutet, oder dessen Salz enthalten, die gestellte Aufgabe in hervorragender Weise gelöst wird.

In den Haarfärbemitteln sollen die erfindungsgemäßen Entwicklersubstanzen, von denen das neue 4-Amino-2-(2'-hydroxyethyl)-phenol und das 4-Amino-2-hydroxymethyl-phenol bevorzugt werden, in einer Konzentration von etwa 0,01 bis 3,0 Gew. %, vorzugsweise 0,1 bis 2,5 Gew. %, enthalten sein.

Obwohl die vorteilhaften Eigenschaften der hier beschriebenen neuen Entwicklersubstanzen es nahelegen, diese als alleinige Entwickler zu verwenden, ist es selbstverständlich auch möglich, die neuen Entwicklersubstanzen der Formel I gemeinsam mit bekannten Entwicklersubstanzen, wie z. B. 1,4-Diaminobenzol, 2,5-Diaminotoluol oder 2,5-Diaminobenzylalkohol, einzusetzen.

Von den bekannten Kupplersubstanzen kommen als Bestandteil der hier beschriebenen Haarfärbemittel vor allem Resorcin, 4-Chlorresorcin, 4,6-Dichlorresorcin, 2-Methylresorcin, 2-Amino-4-(2'-hydroxyethylamino)-anisol, 2,4-Diaminobenzylalkohol, 2,4-Diamino-phenylethylalkohol, m-Phenylendiamin, 5-Amino-2-methyl-phenol, 2,4-Diaminophenoxyethanol, 4-Amino-2-hydroxyphenoxyethanol, 1-Naphthol, m-Aminophenol, 3-Amino-2-methylphenol, m-Toluylendiamin, 4-Hydroxy-1,2-methylendioxybenzol, 4-Amino-1,2-methylendioxybenzol, 4-(2'-Hydroxyethylamino)-1,2-methylendioxybenzol, 2,4-Diaminoanisol, 2,4-Diaminophenetol, 4-Hydroxyindol, 3-Amino-5-hydroxy-2,6-dimethoxypyridin und 3,5-Diamino-2,6-dimethoxypyridin in Betracht.

Die genannten Kuppler- und Entwicklersubstanzen können in den Haarfärbemitteln jeweils einzeln oder im Gemisch miteinander enthalten sein.

Die Gesamtmenge der in den hier beschriebenen Haarfärbemitteln enthaltenen Entwicklersubstanz-Kupplersubstanz-Kombination soll etwa 0,1 bis 5,0 Gew. %, vorzugsweise 0,5 bis 4,0 Gew. %, betragen.

Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponente diesbezüglich in einem gewissen Überschuß oder Unterschuß vorhanden ist.

Weiterhin können die Haarfärbemittel dieser Anmeldung zusätzlich andere Farbkomponenten, beispielsweise 6-Amino-2-methyl-phenol und 2-Amino-5-methyl-phenol, sowie ferner übliche direktziehende Farbstoffe, zum Beispiel Triphenylmethanfarbstoffe wie Diamond Fuchsine (C.I. 42 510) und Leather Ruby HF (C.I. 42 520), aromatische Nitrofarbstoffe wie 2-Nitro-1,4-diamino-benzol, 2-Amino-4-nitro-phenol, 2-Amino-5-nitro-phenol, 2-Amino-4,6-dinitro-phenol, 2-Amino-5-(2'-hydroxyethylamino)-nitrobenzol und 2-Methylamino-5-bis-(2'-hydroxyethyl)-amino-nitrobenzol, Azofarbstoffe wie Acid Brown 4 (C.I. 14 805) und Dispersionsfarbstoffe, wie beispielsweise 1,4-Diaminoanthrachinon und 1,4,5,8-Tetraamino-anthrachinon, enthalten. Die Haarfärbemittel können diese Farkomponenten in einer Menge von etwa 0,1 bis 4,0 Gew. % enthalten.

Selbstverständlich können die Kuppler- und Entwicklersubstanzen sowie die anderen Farbkomponenten, sofern es Basen sind, auch in Form der physiologisch verträglichen Säureadditionssalze, wie beispielsweise als Hydrochlorid bzw. Sulfat oder - sofern sie aromatische OH-Gruppen besitzen - in Form der Salze mit Basen, zum Beispiel als Alkaliphenolate, eingesetzt werden.

Darüber hinaus können in den Haarfärbemitteln noch weitere übliche kosmetische Zusätze, beispielsweise Antioxidantien wie Ascorbinsäure, Thioglykolsäure oder Natriumsulfit, Parfümöle, Komplexbildner, Netzmittel, Emulgatoren,

Verdicker, Pflegestoffe und andere vorhanden sein.

Die Zubereitungsform kann beispielsweise eine Lösung, insbesondere eine wäßrige oder wäßrig-alkoholische Lösung, sein. Die besonders bevorzugten Zubereitsungsformen sind jedoch eine Creme, ein Gel oder eine Emulsion.

Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol, oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gew. %.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin , oder auch anorganische Basen wie Natriumhydroxid und Kaliumhydroxid, Verwendung finden.

Für die Anwendung zur oxidativen Färbung von Haaren vermischt man die vorstehend beschriebenen Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form der 3 bis 12 %igen, vorzugsweise 6 %igen, wäßrigen Lösungen, in Betracht. Wird eine 6 %ige Wasserstoffperoxidlösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50°C etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die Herstellung der erfindungsgemäß verwendeten Entwicklersubstanzen ist zum Teil bekannt. So ist zum Beispiel die Herstellung von 4-Amino-2-hydroxymethyl-phenol in der deutschen Patentschrift 148 977 beschrieben. Sofern ihre Herstellung nicht beschrieben ist, können die Entwicklersubstanzen der Formel I nach dem nachfolgenden Reaktionsschema hergestellt werden:

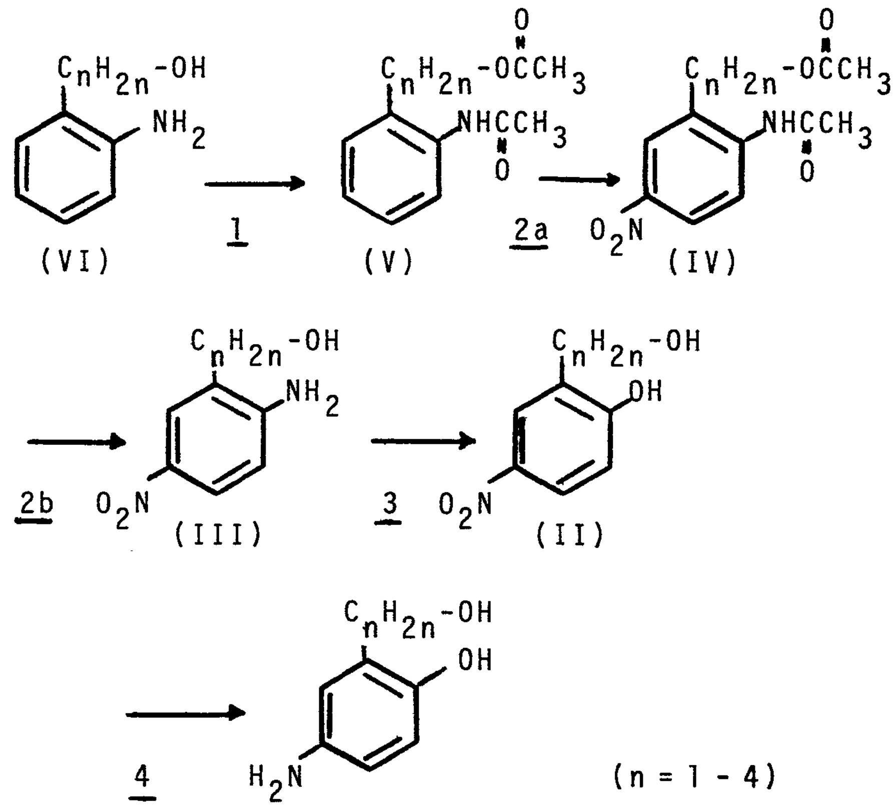


(n = 1 - 4)

Ausgehend von 2-Hydroxyalkyl-anilin (VI), werden zunächst die Alkoholgruppe und die Aminogruppe durch Umsetzung mit Essigsäureanhydrid geschützt (Reaktionsschritt 1). Die entstandene Diacetylverbindung (V) wird anschließend nitriert (Reaktionsschritt 2a) und das Reaktionsprodukt durch Erwärmen mit verdünnter Salzsäure zum 4-Nitro-2-hydroxyalkyl-anilin (III) verseift (Reaktionsschritt 2b). Durch Diazotierung der Verbindung III und anschließende Diazoniumsalzverkochung wird das 4-Nitro-2-hydroxyalkyl-phenol (II) erhalten (Reaktionsschritt 3). Schließlich gelangt man durch katalytische Reduktion der Nitrogruppe der Nitroverbindung (II) mit Wasserstoff am Platinkatalysator zu dem gewünschten 4-Amino-2-hydroxyalkyl-phenol.

4-Amino-2-(2'-hydroxyethyl)-phenol kann ferner durch Nitrierung oder Nitrosierung von 2-Hydroxy-phenylethylalkohol und anschließende Reduktion der Nitrogruppe hergestellt werden.

Die Salze der Verbindungen der Formel I sind durch Umsetzung mit organischen oder anorganischen Säuren oder Basen erhältlich.

Die Entwicklersubstanzen der Formel I sollen in den Haarfärbemitteln entweder als freie Basen oder in Form ihrer physiologisch verträglichen Salze mit anorganischen oder organischen Säuren, zum Beispiel als Chlorid, Sulfat, Phosphat, Acetat, Propionat, Lactat oder Citrat, eingesetzt werden. Im stark alkalischen Medium können sie ferner als Phenolat vorliegen. Die Verbindungen der Formel I sind gut in Wasser löslich, sie weisen außerdem eine ausgezeichnete Lagerstabilität, insbesondere als Bestandteil der hier beschriebenen Haarfärbemittel, auf.

Die erfindungsgemäßen Haarfärbemittel auf der Basis der 4-Amino-2-hydroxyalkyl-phenole als Entwicklersubstanz führen zu Haarfärbungen mit ausgezeichneten Echtheitseigenschaften, insbesondere was die Licht-, Wasch- und Reibechtheit anbetrifft, und lassen sich mit Reduktionsmitteln wieder abziehen.

Von besonderer Bedeutung ist weiterhin der durch die Verwendung der 4-Amino-2-hydroxyalkyl-phenole in den Haarfärbemitteln gemäß vorliegender Anmeldung in toxikologischer und dermatologischer Hinsicht erzielte Fortschritt im Vergleich zu bekannten, für die Erzielung von Rottönen gebräuchlichen Entwicklersubstanzen.

Hinsichtlich der färberischen Möglichkeiten bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre gute Farbintensität aus.

Dies wird insbesondere deutlich beim Vergleich von Haarfärbemitteln, die als Entwicklersubstanz einerseits die bekannten Entwickler p-Aminophenol, 4-Amino-2-methylphenol oder 4-Amino-3-methylphenol und andererseits das anmeldungsgemäße 4-Amino-2-hydroxymethyl-phenol enthalten.

Während das 4-Amino-2-methyl-phenol im Vergleich zum Standard p-Aminophenol schwächere und blauere Färbungen liefert, führt das 4-Amino-2-hydroxymethyl-phenol zu etwa gleichen Farbtönen und vergleichbarer Farbtiefe. Das 4-Amino-3-methyl-phenol, das eine ebenfalls strukturell ähnliche Verbindung darstellt, ergibt erheblich geringere Farbtiefen als die erfindungsgemäßen 4-Amino-2-hydroxyalkyl-phenole.

Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

In den nachstehenden Beispielen soll der Gegenstand der Erfindung näher erläutert werden.

HERSTELLUNGSBEISPIEL

Beispiel 1

Herstellung von 4-Amino-2-(2'-hydroxyethyl)-phenol

Stufe 1: Diacetylverbindung des 2-Aminophenylethylalkohols

100,0 g (0,73 mol) 2-Aminophenylethylalkohol werden in 400 ml (4,23 mol) Essigsäureanhydrid 1 Stunde lang auf 80°C erwärmt. Anschließend läßt man das Gemisch abkühlen und gießt es auf Wasser. Die ausgefallene Diacetylverbindung wird durch Filtrieren abgetrennt. Nach dem Trocknen erhält man 125 g (78 % der Theorie) farblose Kristalle vom Schmelzpunkt 103°C.

Stufe 2: 2-Amino-5-nitrophenylethylalkohol

110,5 g (0,50 mol) der Diacetylverbindung aus Stufe 1 werden bei -10°C in 375 ml konzentrierter Schwefelsäure gelöst und bei -5 bis 0°C mit 36 ml (0,87 mol) Salpetersäure (d = 1,5) in 285 ml konzentrierter Schwefelsäure nitriert. Nach beendeter Reaktion wird auf Eis gegossen und von der Mischung der 4- und 5-Nitrodiacetylverbindung abgesaugt.

Zur Trennung der p-Nitroverbindung von der m-Nitroverbindung wird die Mischung beider Diacetyl-Nitroverbindungen durch Erwärmen mit halbkonzentrierter wäßriger Salzsäurelösung verseift. Nach der Abspaltung der Acetylgruppen verdünnt man mit Wasser und läßt abkühlen. Vom ausgefallenen Niederschlag wird die Flüssigkeit abgesaugt und der

Rückstand mit Wasser gerührt. Dabei wird das Hydrochlorid der p-Verbindung hydrolysiert. Die p-Verbindung fällt in fester Form an und kann von der Lösung des Hydrochlorids der m-Verbindung durch Filtration abgetrennt werden. Die freie Base des 2-Amino-5-nitrophenylethylalkohols wird aus Chloroform umkristallisiert und schmilzt dann bei 108°C.

Ausbeute: 10 g (8 % der Theorie) an 5-Nitroverbindung, die 4-Nitroverbindung entsteht in etwa 8-facher Menge.

Stufe 3: 5-Nitro-2-hydroxyphenylethylalkohol

3,3 g (18 mmol) des 2-Amino-5-nitrophenylethylalkohols aus Stufe 2 werden in 50 ml 35 %iger Schwefelsäure gelöst und mit 1,36 g (20 mmol) Natriumnitrit in 5 ml Wasser bei 0°C diazotiert.

Die Diazoniumsalzlösung wird anschließend filtriert und in heiße 50 %ige Schwefelsäure gegossen. Man erwärmt das Gemisch auf 100°C und läßt es sodann abkühlen. Der 5-Nitro-2-hydroxyphenylethylalkohol fällt aus und wird zur Abtrennung von Nebenprodukten aus Toluol umkristallisiert. Man erhält gelbe Kristalle vom Schmelzpunkt 157°C.

Stufe 4: 4-Amino-2-(2'-hydroxyethyl)-phenol

Die Nitroverbindung der Stufe 3 wird in Ethanol katalytisch mit Wasserstoff an Platin bei Normaldruck und Raumtemperatur reduziert. Nach Aufnahme der theoretischen Menge Wasserstoff wird vom Katalysator abfiltriert und das Lösungsmittel im Vakuum verdampft. Man erhält das 4-Amino-2-(2'-hydroxyethyl)-phenol in Form leicht rosafarbener Kristalle vom Schmelzpunkt 94°C.

BEISPIELE FÜR HAARFÄRBEMITTEL

Beispiel 2 Haarfärbemittel in Gelform

| | |
|---|---|
| 0,50 g | 4-Amino-2-hydroxymethyl-phenol |
| 1,00 g | 2-Amino-4-(2'-hydroxyethyl)-amino-anisolsulfat |
| 0,15 g | Natriumsulfit, wasserfrei |
| 5,00 g | Laurylalkohol-diglykolethersulfat-Natriumsalz (28 %ige wäßrige Lösung) |
| 1,00 g | Hydroxyethylcellulose, hochviskos |
| 10,00 g | Ammoniak, 22 %ig |
| 82,35 g | Wasser |
| 100,00 g | |

50 g des obigen Haarfärbemittels werden kurz vor dem Gebrauch mit 50 g Wasserstoffperoxidlösung (6 %ig) gemischt und das Gemisch anschließend auf blonde Naturhaare aufgetragen. Nach einer Einwirkungszeit von 30 Minuten bei 40°C wird mit Wasser gespült und getrocknet. Das Haar hat eine intensive leuchtend rote Färbung mit Violettstich erhalten.

Beispiel 3 Haarfärbemittel in Gelform

| | |
|---|---|
| 0,35 g | 4-Amino-2-(2'-hydroxyethyl)-phenol |
| 0,27 g | 5-Amino-2-methylphenol |
| 0,30 g | Ascorbinsäure |
| 15,00 g | Ölsäure |
| 7,00 g | Isopropanol |
| 10,00 g | Ammoniak, 22 %ig |
| 67,08 g | Wasser |
| 100,00 g | |

Man vermischt kurz vor dem Gebrauch 50 g dieses Haarfärbemittels mit 50 g Wasserstoffperoxidlösung (6 %ig) und läßt das Gemisch 30 Minuten lang bei 40°C auf weiße menschliche

Haare einwirken. Sodann wird mit Wasser gespült und getrocknet. Das Haar ist in einem orangen Farbton gefärbt.

Beispiel 4 Haarfärbemittel in Cremeform

| | |
|---|---|
| 1,00 g | 4-Amino-2-hydroxymethyl-phenol |
| 1,10 g | 1-Naphthol |
| 15,00 g | Cetylalkohol |
| 0,30 g | Natriumsulfit, wasserfrei |
| 3,50 g | Laurylalkohol-diglykolethersulfat-Natriumsalz (28 %ige wäßrige Lösung) |
| 3,00 g | Ammoniak, 22 %ig |
| 76,10 g | Wasser |
| 100,00 g | |

50 g dieses Haarfärbemittels werden kurz vor dem Gebrauch mit 50 g Wasserstoffperoxidlösung (6 %ig) vermischt. Anschließend trägt man das Gemisch auf blonde Naturhaare auf und läßt es 30 Minuten lang bei 40°C einwirken. Danach wird mit Wasser gespült und getrocknet. Das Haar hat eine intensive Rotfärbung erhalten.

Beispiel 5 Haarfärbelösung

| | |
|---|---|
| 0,80 g | 4-Amino-2-hydroxymethyl-phenol |
| 0,12 g | Resorcin |
| 0,10 g | m-Aminophenol |
| 0,50 g | 5-Amino-2-methylphenol |
| 0,10 g | 2,4-Diaminoanisolsulfat |
| 0,05 g | 1-Naphthol |
| 10,00 g | Laurylalkohol-diglykolethersulfat-Natriumsalz (28 %ige wäßrige Lösung) |
| 10,00 g | Ammoniak, 22 %ig |
| 78,33 g | Wasser |
| 100,00 g | |

Man vermischt kurz vor dem Gebrauch 50 g des vorstehenden Haarfärbemittels mit 50 g Wasserstoffperoxidlösung (6 %ig) und läßt die Mischung 30 Minuten lang bei 40°C auf blonde Naturhaare einwirken. Sodann wird mit Waaser gespült und getrocknet. Das Haar ist in einem modischen goldorangen Farbton gefärbt.

Beispiel 6: Färbemittel in Gelform

| | |
|---|---|
| 0,90 g | 4-Amino-2-hydroxymethyl-phenol |
| 0,80 g | 2,5-Diaminotoluolsulfat |
| 0,15 g | 2,4-Diaminoanisolsulfat |
| 0,20 g | 5-Amino-2-methylphenol |
| 0,02 g | 1-[(2'-Ureidoethyl)-amino] -4-nitrobenzol |
| 0,05 g | 2-Nitro-p-phenylendiamin |
| 0,15 g | Natriumsulfit, wasserfrei |
| 2,50 g | Laurylalkohol-diglykolethersulfat-Natrium-salz (28 %ige wäßrige Lösung) |
| 0,80 g | Hydroxyethylcellulose, hochviskos |
| 6,00 g | Ammoniak, 22 %ig |
| 88,43 g | Wasser |
| 100,00 g | |

50 g des obigen Haarfärbemittels werden kurz vor dem Gebrauch mit 50 g Wasserstoffperoxidlösung (6 %ig) gemischt und die Mischung anschließend auf blonde Naturhaare aufgebracht. Nach einer Einwirkungszeit von 30 Minuten bei 40°C wird mit Wasser gespült und getrocknet. Das Haar hat eine modische braune Färbung erhalten.

Alle in der vorliegenden Anmeldung angegebenen Prozentzahlen stellen Gewichtsprozente dar.

## Patentansprüche

1. Mittel zur oxidativen Färbung von Haaren auf der Basis einer Entwicklersubstanz-Kupplersubstanz-Kombination, dadurch gekennzeichnet, daß es als Entwicklersubstanz ein 4-Amino-2-hydroxyalkyl-phenol der allgemeinen Formel I

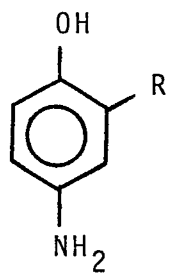

(I),

wobei R einen Monohydroxyalkylrest mit 1 bis 4 Kohlenstoffatomen bedeutet, oder dessen Salz enthält.

2. Mittel nach Anspruch 1, dadurch gekennzeichnet, daß als 4-Amino-2-hydroxyalkyl-phenol das 4-Amino-2-hydroxymethyl-phenol, 4-Amino-2-(2'-hydroxyethyl)-phenol oder das Salz dieser Verbindung enthalten ist.

3. Mittel nach Anspruch 1 und 2, dadurch gekennzeichnet, daß die Kupplersubstanz ausgewählt ist aus 1-Naphthol, Resorcin, 4-Chlorresorcin, 4,6-Dichlorresorcin, 2-Methylresorcin, 2-Amino-4-(2'-hydroxyethylamino)-anisol, 5-Amino-2-methyl-phenol, 2,4-Diaminophenoxyethanol, 4-Amino-2-hydroxy-phenoxyethanol, m-Aminophenol, 3-Amino-2-methylphenol, 4-Hydroxy-1,2-methylendioxybenzol, 4-Amino-1,2-methylendioxybenzol, 4-(2'-Hydroxyethylamino)-1,2-methylendioxybenzol, 2,4-Diaminoanisol, 2,4-Diaminophenetol, 2,4-Diaminobenzylalkohol, m-Phenylendiamin, m-Toluylendiamin, 2,4-Diamino-phenylethylalkohol, 4-Hydroxyindol, 3-Amino-5-hydroxy-2,6-dimethoxypyridin und 3,5-Diamino-2,6-dimethoxypyridin.

4. Mittel nach Anspruch 1 bis 3, dadurch gekennzeichnet, daß die Entwicklersubstanz der Formel I in einer Menge von 0,01 bis 3,0 Gewichtsprozent enthalten ist.

5. Mittel nach Anspruch 1 bis 4, dadurch gekennzeichnet, daß die Gesamtmenge der Entwickler-Kupplersubstanz-Kombinationen 0,1 bis 5,0 Gewichtsprozent, vorzugsweise 0,5 bis 4,0 Gewichtsprozent, beträgt.

6. Mittel nach Anspruch 1 bis 5, dadurch gekennzeichnet, daß es eine Farbkomponente enthält, welche ausgewählt ist aus 6-Amino-2-methyl-phenol, 2-Amino-5-methyl-phenol, Diamond Fuchsine (C.I. 42 510), Leather Ruby HF (C.I. 42 520), 2-Nitro-1,4-diamino-benzol, 2-Amino-4-nitro-phenol, 2-Amino-5-nitro-phenol, 2-Amino-4,6-dinitro-phenol,2-Amino-5-(2'-hydroxyethylamino)-nitrobenzol, 2-Methylamino-5-bis-(2'-hydroxyethyl)-amino-nitro-benzol, Acid Brown 4 (C.I. 14 805), 1,4-Diaminoanthrachinon und 1,4,5,8-Tetraamino-anthrachinon.

7. Mittel nach Anspruch 1 bis 6, dadurch gekennzeichnet, daß es Antioxidantien, vorzugsweise Ascorbinsäure oder Natriumsulfit, enthält.

8. Mittel nach Anspruch 1 bis 7, dadurch gekennzeichnet, daß es einen pH-Wert zwischen 8,0 und 11,5 aufweist.

9. 4-Amino-2-(2'-hydroxyethyl)-phenol.

0182187

Nummer der Anmeldung

Europäisches Patentamt

# EUROPÄISCHER RECHERCHENBERICHT

EP 85 11 4018

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| | | | A 61 K 7/13 |
| D,Y | DE-C- 148 977 (FARBENFABRIKEN VORM. F. BAYER & CO.)<br>* Insgesamt *<br>--- | 1,2 | |
| Y | DE-A-2 447 017 (HENKEL & CIE GmbH)<br>* Seite 5, Zeile 15; Ansprüche *<br>--- | 1-9 | |
| Y | EP-A-0 043 436 (WELLA AG)<br>* Seite 4, Zeile 21 - Seite 7, Zeile 6; Ansprüche 1,4-9 *<br>--- | 3-8 | |
| Y | GB-A-2 026 553 (L'OREAL)<br>* Seite 4, Zeilen 69-81; Ansprüche *<br>----- | 1-9 | |
| | | | RECHERCHIERTE SACHGEBIETE (Int Cl 4) |
| | | | A 61 K<br>C 07 C<br>D 06 P |

Der vorliegende Recherchenbericht wurde für alle Patentansprüche erstellt.

| Recherchenort | Abschlußdatum der Recherche | Prüfer |
|---|---|---|
| DEN HAAG | 17-02-1986 | BERTE M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82